# FASCICULE DE BREVET EUROPEEN

(11) **EP 2 333 020 B1**
(45) Date de publication et mention de la délivrance du brevet: **15.05.2013**
(21) Numéro de dépôt: 10014973.1
(22) Date de dépôt: 25.11.2010
(51) Int. Cl.: C09C 1/00, C09C 1/24, C09C 1/36, A61K 8/34, A61K 8/39, A61Q 1/12

(54) **Matiere colorante utilisable notamment dans l'industrie des cosmetiques**
Farbiges Material, insbesondere einsetzbar in der kosmetischen Industrie
Coloured material useable in particular in cosmetic industry

(30) Priorité: 25.11.2009 FR 0905661
(43) Date de publication de la demande: 15.06.2011
(73) Titulaire: S.A. Color, 76380 Canteleu (FR)
(72) Inventeur: Boudin, Michel, 76480 Ambourville (FR); Boudin, Philippe, 76000 Rouen (FR); Leroux, Stéphane, 76370 Berneval Le Grand (FR)
(74) Mandataire: Thibon, Norbert

(56) Documents cités:
- EP-A1- 1 518 903
- EP-A2- 0 323 407
- GB-A- 896 067

## Description

La présente invention concerne le domaine des matières colorantes à base de pigments pulvérulents enrobés dans un liant organique qui se présentent à l'état solide sous forme aisément dispersible dans l'eau. Elle concerne plus spécialement les matières colorantes utilisables dans la fabrication de cosmétiques.

Dans le domaine des cosmétiques, on propose de nombreux produits dont la composition comporte une matière colorante. lls se présentent sous forme de poudres, sèches ou grasses, de liquides, de pâtes plus ou moins consistantes, qui peuvent être sous forme d'émulsions huileuses, de gels aqueux, ou de crèmes, et qui sont destinés à être appliqués sur la peau, telle que celle des lèvres, sur des muqueuses, ou sur des poils, tels que ceux des cils, de la barbe, ou des cheveux, que ce soit dans un simple but de maquillage ou pour tout autre soin.

Dans la fabrication de tels produits, l'industrie cosmétiques rencontre de sérieuses difficultés à utiliser des colorants ou pigments qui sont au départ insolubles. Il s'avère alors nécessaire de les broyer et de les rendre mouillables pour les intégrer à des compositions à phase aqueuse, ce qui fait appel à des moyens d'agitation, de chauffage, de mise en pression. Outre que ces moyens sont gros consommateurs d'énergie, ils ont l'inconvénient de devoir être mis en oeuvre au cours de la procédure de fabrication, ce qui limite l'étendue des gammes de couleurs proposées dans les produits commercialisés. En pratique, il n'est pas possible actuellement d'adapter la teinte d'un produit cosmétique à un client particulier afin d'obtenir la couleur ou nuance qui, par exemple conviendra le mieux à sa carnation, ou afin de créer des effets coloriels ou optiques particuliers.

L'industrie cosmétique est donc en demande pour des matières colorantes qu'il lui soit plus facile d'utiliser dans la fabrication de ses produits sans perturber leurs qualités cosmétiques et qui permettent, le cas échéant, de commercialiser des préparations cosmétiques dont la teinte puisse encore être adaptée en fonction de l'utilisateur final.

L'invention vise à satisfaire à cette demande, en proposant des matière colorantes à base de pigments enrobés dans un liant organique dont la composition répond aux besoins de l'industrie cosmétique. notamment en ce qui concerne le respect des qualités propres qui sont recherchées pour des produits de maquillage ou de soin de la peau, la facilité de mise en dispersion ou psaudo-solutlon de pigments de dimensions microniques ou sub-microniques, la stabilité et l'homogénèite des dispersions obtenues en milieu aqueux à partir de pigments pulvérulents insolubles.

Elle vise aussi à proposer de telles matières qui, par leur forme soient faciles d'emploi dans les conditions de fabrication des produits cosmétiques, en particulier par la rapidité de mise en suspension sous forme homogène et stable et par le fait qu'elles ne sont pas salissantes comme le seraient les pigments commercialisés sous forme pulvérulente.

L'invention vise encore à proposer de telles matières colorantes qui présentent un pH proche de la neutralité afin de ne pas altérer la peau et dont la présence dans des formulations de produits cosmétiques préserve les qualités sensorielles propres à ces produits cosmétiques et favorise un fort pouvoir couvrant et/ou un fort pouvoir coloriel, grâce notamment à un taux élevé de pigments pour une faible proportion de liant orgnique dans la composition globale.

Dans un tel contexte, la présente invention a pour objet une matière colorante essentiellement constituée de pigments pulvérulents enrobés dans un liant organique dans laquelle ledit liant est constitué ce sorbitol et dont la composition comprend, en pourcentage pondéral dans le poids total de la composition, de 60 à 95 % en poids de pigments pulvérulents pour 1 à 20 % de sorbitol, ainsi que les constituants en proportions stoechiométriques d'un agent à effet effervescent en présence d'eau et qu'un agent dispersant.

La proportion de liant organique est ainsi largement minoritaire, par rapport à la proportion de pigment. Ceci a le double avantage pour les applications en cosmétologie de conduire à un bon pouvoir couvrant en évitant les risques de phénomènes d'agglomération qui nuiraient a l'uniformité de la couche du produit cosmétique une fois appliqué sur la peau. De ce point de vue, les proportions préférées sont de 70 à 90 % en poids de pigment et de 2 à 10 % en poids de sorbitol dans le poids total de la composition globale.

La matière colorante répondant à une telle composition est produite sous forme de granulés. Cette présentation facilite le transport de la matière ainsi que sa mise en oeuvre dans ses applications en fabrication de produits cosmétiques, les granulés étant à l'état solide à la température ordinaire. Les dimensions des granulés sont avantageusement comprises entre 0,1 mm et 5 mm, et de préférence de l'ordre de 0,5 à 2 mm.

Dans la composition suivant l'invention, la présence d'un agent effervescent et d'un agent dispersant en combinaison permet d'obtenir a dispersibilité souhaitable malgré la proportion largement prépondérante nu pigment à disperser par rapport au liant soluble dans l'eau, et ce tout en évitant qu'une quantité trop importante d'agent effervescent producteur de mousse vienne perturber l'emploi de la matière colorante dans le cadre de la fabrication d'un produit cosmétique ou que sa présence provoque la formation de bulles lors de l'application du produit cosmétique, dans la mesure où l'agent dispersant n'implique pas les mêmes risques.

L'invention propose ainsi une matière colorante, qui se présente préférentiellement sous forme de grains individualisés, donc de granules, à l'état solide dans les conditions normales de température et pression, dans laquelle des particules de pigments pulvérulents, notamment de pigments minéraux-reconnus utilisables dans le domaine des cosmétiques sont enrobées et liées, avec un agent effervescent et un agent dispersant, dans un liant d'enrobage hydrosoluble, solide à la température ordinaire (25°C) et sous la pression atmosphérique, mais fusible à une température plus élevée, restant cependant inférieur à celle de l'ébullition de l'éau.

Comme agent d'enrobage, le choix du sorbitol est particulièrement avantageux. Il s'agit d'un produit non toxique, qui fond vers 95°C, donc à une température assez basse pour être facile à atteindre, mais suffisamment haute pour rester solide même quand la température ambiante s'élève à plus de 25°C comme cela se produit en été. Le sorbitol est un polyol très hydrosoluble à température et pression normales. Il se dissout en présence d'humidité, ce qui donne un certain pouvoir collant à la matière colorante et lui permet de bien adhérer à la peau quand elle est incorporée dans une formulation cosmétique. Par ailleurs, il est possible de broyer ultérieurement la matière colorante ainsi constituée sans générer de poussière. Le sorbitol est également favorable en vue d'une dispersibilité rapide et importante des pigments lorsque la matière colorante est mise dans l'eau.

L'invention vise plus spécialement les produits à base de pigments minéraux car ils sont bien adaptés à une utilisation en cosmétique, du fait qu'ils sont en général stables à la lumière et à l'air et qu'ils sont peu allergisants. Selon l'invention, les pigments sont de préférence des pigments minéraux. Ils sont plus particulièrement choisis parmi les oxydes de fer, le dioxyde de titane, les pigments à effet optique, notamment à effet interférentiel (ou iridescent) de type mica-oxyde de titane, et leurs mélanges. Comme oxydes de fer, on peut utiliser notamment les oxydes de fer jaune, rouge ou noir. Comme pigments on peut aussi utiliser des pigments à effet métallique.

L'invention propose plus précisément une matière colorante, dispersible en milieu aqueux, se présentant sous forme divisée telle que des granulés solides, constituée essentiellement de pigments en mélange avec un agent effervescent et un dispersant; enrobés et liés avec du sorbitol et telle qu'elle comprenne, en poids par rapport au poids total de la matière, entre 60 et 95 % de pigments (de préférence plus de 90 %) et entre 1 et 20 % de sorbitol. Les pigments, l'agent effervescent, l'agent dispersant ont été préalablement mis en mélange avec le sorbitol à une température supérieure ou égale à une température permettant la fusion du sorbitol qui lie et enrobe ces constituants.

Dans le cas d'une utilisation de la matière colorante en cosmétique, les ingrédients de la matière doivent évidemment être choisis parmi ceux autorisés en cosmétique.

Les quantités des constituants exprimées en pourcentage, ci-dessus et dans la suite de la description sauf indication contraire, sont bien évidemment choisies dans les fourchettes indiquées de manière à ce que la somme des quantités pour tous les constituants de la matière soit égale à 100.

L'agent effervescent peut présenter une certaine acidité. Afin de respecter la neutralité de la peau dans le cas d'utilisation pour des cosmétiques, sa quantité sera de préférence inférieure à 20% en poids. De plus une quantité relativement faible évite d'avoir trop de bulles lorsque la réaction effervescente se produit et de générer de la mousse indésirable. De préférence la matière colorante anhydre selon l'invention, comprend de 1 à 20 % d'agent effervescent, en poids par rapport au poids total de la matière.

Le dispersant confère à la matière colorante la propriété d'être dispersible en milieu aqueux et plus spécialement en milieu aqueux gras (eau+huile) pour réaliser notamment des émulsions ou des crèmes grasses. Le dispersant et l'agent effervescent agissent de manière complémentaire pour rendre la matière colorante dispersible en milieu aqueux, notamment en milieu aqueux gras.

Le dispersant est un composé tensio-actif connu. Il est de préférence choisi parmi les alcools gras éthoxylés, y compris ceux à chaîne ramifiée Selon l'invention, la matière colorante comprend de préférence un dispersant choisi parmi les alcools gras éthoxylés, en une quantité comprise entre 0,1 et 20% en poids, par rapport au poids total de la matière.

Selon un cas particulier de l'invention et préféré en cosmétique l'alcool gras éthoxylé est issu d'un alcool gras comportant entre 16 et 19 atomes de carbone. En particulier l'alcool gras éthoxylé est un éther oxyéthyléné de l'alcool cétéarylique, alcool à 16-18 atomes de carbone. Plus particulièrement ce dernier comporte 25 groupes éthoxy.

La matière colorante selon l'invention comprend de préférence de 70 à 90 % de pigments, en poids par rapport au poids total de la matière.

De préférence aussi, elle comprend de 2 à 10 % de sorbitol, de préférence encore de 4 à 8%, en poids par rapport au poids total de la matière.

Elle comprend de préférence de 5 à 15 % d'agent effervescent, en particulier de 8 à 12%, en poids par rapport au poids total de la matière.

De préférence aussi, la matière colorante suivant l'invention comprend de 0,5 à 10% du dispersant choisi parmi les alcools gras éthoxylés, en particulier de 1 à 5%.

De préférence selon l'invention, l'agent effervescent est uni mélange d'acide citrique et de carbonate de sodium ou de bicarbonate de sodium mis en proportions stoechiométriques pour donner lieu en présence d'eau à la réaction effervescente qui dégage du dioxyde de carbone. Comme (bi)carbonates, on peut utiliser des sels d'alcalino-terreux autres que le sodium, notamment de calcium. Toutefois les sels de sodium sont préférés car ils présentent une grande solubilité danse l'eau qui confère une dispersibilité appréciable des pigments lorsque la matière colorante est mise en présence d'eau.

Selon l'invention, la matière colorante est sous forme de granulés qui ont de préférence une taille moyenne comprise entre 0,1 et 5 mm. De préférence encore la granulométrie moyenne est comprise entre 0,5 et 2 mm.

La matière colorante selon l'invention est utilisée de préférence en cosmétique en milieu industriel pour réaliser des produits cosmétiques, qui sont présentés notamment en poudre ou émulsion dans des crèmes ou dans des gels aqueux. Elle peut aussi être utilisée par le consommateur pour finaliser un produit cosmétique à appliquer sur la peau, notamment pour en régler la teinte.

Les poudres cosmétiques comportent des additifs qui apporte une humidité suffisante pour disperser les pigments de la matière colorante. Le cas échéant, de l'eau ou une base cosmétique hydratante est préalablement appliquée sur la peau.

La matière colorante peut être mélangée avec une composition comprenant de l'eau et éventuellement une matière grasse (huileuse) juste avant son utilisation par les utilisateurs finaux de manière à obtenir la teinte ou effet optique qu'ils souhaitent, et également selon la quantite souhaitée.

L'invention concerne aussi le procédé de fabrication d'une matière colorante, se présentant sous forme de granulés solides, telle que décrite précédemment.

Ce procédé de fabrication comprend les étapes suivantes :
- on prépare au préalable l'agent effervescent par mélange d'acide citrique et de (bi)carbonate de sodium sous forme cristalline, en proportions stoechiométriques,
- on. mélange à sec les pigments avec l'agent effervescent, et un argent dispersant choisi parmi les alcools gras éthoxylés, et du sorbitol,
- on chauffe ce mélange à une température d'environ 100°C pour fondre le sorbitol, puis
- on laisse refroidir l'ensemble en le soumettant à une agitation modérée jusqu'à complet refroidissement pour éviter l'agglomération en blocs,
- on tamise les granulés obtenus à la granulométrie requise.

L'invention sera maintenant plus avant précisée dans ses caractéristiques préférées par les exemples de mise en oeuvre ci-après. concernant la formulation et la préparation de compositions colorantes anhydres à base de pigments minéraux qui sont destinées à servir à la coloration de produits cosmétiques,

Sauf stipulation contraire, toutes les grandeurs chiffrées ou autres indications seront exprimées en conformité avec la normalisation internationale d'une part, en quantités massiques d'autre part.

### Préparation des granulés

Chacun des produits dont la composition est indiquée dans le tableau 1 est obtenu sous forme de granulés par un procédé de granulation à l'état fondu, en opérant de la façon décrite ci-après. L'invention y est appliquée à la préparation de matières colorantes à base de pigments minéraux pulvérulents (granulométrie sub-micronique) d'oxydes de fer ou de dioxyde de titane.

Plus spécialement, pour les oxydes de fer il s'agit de pigments commercialisés par la société Sun Chemical, qui portent chez ce fabricant la référence C33-9001 pour l'oxyde de fer jaune, la référence C33-8001 pour l'oxyde de fer rouge, la référence C33-7001 pour l'oxyde de fer noi-. Pour chacun; la taille de particules de pigments est inférieure à 1 µm. La taille moyenne est respectivement de 0.80 µm, 0,10 µm et 0,15 µ.m. La surface spécifique est comprise entre 10 et 15 m²/g.

Quant au pigment de dioxyde de titane employé dans les exemples, il s'agit de celui qui est commercialisé sous le nom HombitanⓇ Anatase FF-Pharma par la société SACHTLEBEN. Dans ce cas, la taille moyenne des particules est d'environ 0,4 µm et la surface spécifique est de 9 m²/g environ.

Les différents constituants sous forme pulvérulente sont mélangés à sec, dans les proportions qui sont indiquées dans le tableau. où elles sont exprimées en pourcentage en poids par rapport au poids total du mélange. L'agent effervescent aura toutefois été préparé au préalable, par mélange d'acide citrique et de carbonate de sodium, l'un et l'autre sous forme cristalline, dans les proportions stoechiométriques de la réaction effervescente entre les deux constituants. La réaction recherchée a lieu en présence d'eau. Elle entraîne la production de dioxyde de carbone, lequel est responsable de l'effet effervescent. En l'absence d'humidité, l'agent effervescent est inerte.

Le dispersant est choisi parmi les alcools gras éthoxylés, de préférence parmi les composés éthoxylés d'alcools gras dont la chaîne carbonée compte de 8 à 24 atomes de carbone, et de préférence encore de 12 à 20 atomes de carbone, dans une chaîne ramifiée, et plus spécialement parmi les alcools gras éthoxylés dont la molécule comporte de 20 à 30 fonctions oxyéthylénées.

L'alcool gras éthoxylé choisi en conformité avec l'invention, tel qu'il est utilisé ici, est constitué par un éther oxyéthyléné de l'alcool cétéarylique (alcool gras en C16-C18), en particulier celui à 25 groupes éthoxy. Ce composé est disponible dans le commerce sous la désignation de Ceteareth-25.

Le mélange anhydre des composés est chauffé au bain-marie, à une température d'environ 100 °C, permettant d'atteindre la température de fusion du sorbitol (95°C), jusqu'à ce que le liant soit complètement liquide. Il est ensuite refroidi de manière à assurer sa précipitation, qui se produit sous forme de granulés solides. Ces granulés sont recueillis et maintenus sous agitation modérée jusqu'à complet refroidissement, afin d'éviter leur agglomération en blocs.

Les granulés sont triés par tamisage à travers un tamis de dimension de maille 1 mm. Plus généralement, la granulométrie finale recherchée pour l'application dans la fabrication de produits cosmétiques envisagée ici est habituellement comprise entre 100 micromètres et 5 millimètres. Elle reste cependant de préférence voisine du millimètre, donc notamment comprise entre 0,5 et 2 mm.

**Tableau 1 : Compositions des exemples 1 à 6**

| **Composé** | **Ex. 1** | **Ex.2** | **Ex.3** | **Ex. 4** | **Ex. 5** | **Ex. 6** |
|---|---|---|---|---|---|---|
| Oxyde de fer jaune | 78 | - | - | - | 60 | 55 |
| Oxyde de fer rouge | - | 80 | - | | 20 | 5 |
| Oxyde de fer noir | - | - | 85 | - | - | |
| Dioxyde de titane | | | | 80 | - | 20 |
| Sorbitol | 6,67 | 6,65 | 4,67 | 6,65 | 6,65 | 6,65 |
| Ceteareth-25 | 3,33 | 3,35 | 2,33 | 3,35 | 3,35 | 3,35 |
| Carbonate de sodium | 4,26 | 3,55 | 2,84 | 3,55 | 3,55 | 3,55 |
| Acide citrique | 7,74 | 6,45 | 5,16 | 6,45 | 6,45 | 6,45 |

En variante des exemples ci-dessus, on a remplacé le carbonate de sodium par du carbonate de calcium, en adaptant en conséquence la proportion d'acide citrique pour respecter les proportions de la relation stoechiométrique. Il reste que le carbonate de sodium, pour la même réaction effervescente par production de gaz carbonique, a l'avantage sur le carbonate de calcium d'une meilleure solubilité dans l'eau, de sorte que tout recours à une chélation préalable devient inutile.

Par contre, le carbonate de sodium peut être en réalité du bicarbonate de sodium, en totalité ou en partie.

Dans d'autres variantes de mise en oeuvre du procédé, on prévoit d'utiliser, comme agent d'enrobage, du xylitol à la place du sorbitol. Le xylitol est un polyol hydrosoluble dont la température de fusion est du même ordre de grandeur que celle du sorbitol, mais il est un peu moins soluble que le sorbitol dans les conditions de mise en oeuvre de la présente invention.

Du point de vue des proportions des différents constituants dans la composition globale, les exemples 1 à 4 ci-dessus illustrent des produits qui, suivant l'invention dans ses modes de mise en oeuvre préférés, répondent à une composition comprenant une teneur en pigment comprise entre 70 et 90 %, une proportion de sorbitol comprise entre 4 et 8%, une proportion d'agent effervescent comprise entre 8 et 12 %, une proportion d'agent dispersant comprise entre 1 et 5%. La composition comprend en général 10 à 20 fois plus de pigment que de sorbitol, en poids.

On remarque aussi que la proportion d'agent dispersant et !a proportion d'agent effervescent sont dans un rapport d'un quart à un tiers,

### Exemples d'utïlisation des granulés :

Lorsque les granulés selon l'un des exemples 1 à 6 sont mélangés à une composition cosmétique comprenant une phase aqueuse. ils se dispersent spontanément à température ambiante tout en assurant un développement coloriel immédiat et optimal. Leur concentration va de % à 20% en poids, plus particulièrement de 5 % à 15 % par rapport au poids total du mélange obtenu.

Selon un premier exemple d'application, on peut ajouter de l'ordre de 12 % en poids d'un mélange des granulés obtenus aux exemples 1 à 4, à une composition de crème de soin réalisée au préalable sous forme huile dans eau par dispersion d'une phase grasse dans une phase aqueuse.

Selon un autre exemple d'application, on peut réaliser une composition sous forme sèche en ajoutant aux granulés selon l'exemple 6, des charges additionnelles usuellement utilisées en cosmétique.

Selon encore un autre exemple d'utilisation des granulés selon l'invention, on réalise un fond de teint les contenant. On réalise d'abord une émulsion eau/huile par dispersion d'une phase aqueuse dans une phase grasse pour faire une composition basique de fond de teint. On ajoute ensuite à l'émulsion ainsi obtenue, des granulés obtenus à l'exemple 2, à raison de 2 parts en poids pour 98 parts d'émulsion. On mélange le tout par agitation pendant une minute environ.

La base du fond de teint évolue dès l'introduction des granulés pour atteindre sa couleur définitive rose beige. Aucune trace de manque d'homogénéité de la matière ou de marque d'uniformité de la teinte n'est décelable.

La description qui précède explique clairement comment l'invention permet d'atteindre les objectifs qu'elle s'est fixés. Elle permet notamment de proposer une matière colorante adaptée à l'industrie des cosmétiques, respectant les qualités requises pour les cosmétiques stable, rapidement dispersible en présence d'eau et conférant un pouvoir couvrant et/ou coloriel important à une composition cosmétique la contenant.

Il ressort néanmoins de ce qui précède que l'invention n'est pas limitée aux modes de mise en oeuvre qui ont été spécifiquement, et qu'elle s'étend au contraire à toute variante passant par le biais de moyens équivalents.

## Revendications

1. Matière colorante pour application cosmétique, se présentant sous forme de granulés solides à température ordinaire, à base de pigments pulvérulents enrobés dans un liant organique constitué de sorbitol, présent dans la proportion de 1 à 20 % en poids pour 60 à 95 % en poids de pigments dans sa composition pondérale totale, celle-ci contenant en outre les constituants en proportions stoechiométriques d'un agent effervescent en présence d'eau et un alcool gras éthoxylé en tant qu'agent dispersant, de sorte que ladite matière colorante est dispersible en milieu aqueux.

2. Matière colorante selon la revendication 1, **caractérisée par le fait que** la composition comprend de 1 à 20 % d'agent effervescent, en poids par rapport au poids total de la matière et de 0,1 à 20% du dispersant, en poids par rapport à son poids total.

3. Matière colorante selon l'une des revendications précédentes, **caractérisée par le fait qu'**elle comprend de 70 à 90 % de pigments, en poids par rapport au poids total de la matière, et de 2 à 10 % de sorbitol, de préférence de 4 à 8%, en poids par rapport au poids total.

4. Matière colorante selon l'une des revendications précédentes, **caractérisée par le fait que** la composition comprend de 5 à 15 % d'agent effervescent, en particulier de 8 à 12%, en poids par rapport au poids total de la matière, et de 0,5 à 10% du dispersant, de préférence 1 à 5%, en poids par rapport au poids total de la matière.

5. Matière colorante selon l'une des revendications précédentes_{;} **caractérisée par le fait que** les pigments sont des pigments minéraux choisis parmi les oxydes de fer, le dioxyde de titane, les pigments interférentiels mica-oxyde de titane, et leurs mélanges, sous forme pulvérulente de granulométrie sub-micronique.

6. Matière colorante selon l'une des revendications précédentes, **caractérisée par le fait que** l'agent effervescent est un mélange d'acide citrique et de (bi)carbonate de sodium en proportions stoechiométriques pour donner lieu à un dégagement effervescent de dioxyde de carbone en présence d'eau.

7. Matière colorante selon l'une des revendications précédentes, **caractérisée par le fait que** le dispersant est choisi parmi les alcools gras éthoxylés, de préférence parmi les composés éthoxylés d'alcools gras dont la chaîne carbonée compte de 8 à 24 atomes de carbone, et de préférence encore de 12 à 20 atomes de carbone, dans une chaîne ramifiée, et plus spécialement parmi les alcools gras éthoxylés dont la molécule comporte de 20 à 30 fonctions oxyéthylénées.

8. Matière colorante selon la revendication précédente, **caractérisée par le fait que** l'alcool gras éthoxylé et un éther oxyéthyléné de l'alcool cétéarylique, en particulier comportant 25 groupes éthoxy.

9. Matière colorante selon l'une des revendications précédentes, **caractérisée par le fait qu'**elle se présente sous forme de granulés solides à la température ordinaire dont la taille moyenne est comprise entre 0,1 et 5 mm, de préférence entre 0,5 et 2 mm.

10. Matière colorante selon la revendication précédente, répondant à une composition comprenant une teneur en pigment comprise entre 70 et 90 %, une proportion de sorbitol comprise entre 4 et 8%, une proportion d'agent effervescent comprise entre 8 et 12 %, une proportion d'agent dispersant comprise entre 1 et 5%.

11. Utilisation d'une matière colorante selon l'une quelconque des revendications 1 à 10 pour la fabrication de compositions de produits cosmétiques par incorporation de ladite matière dans lesdites compositions en milieu aqueux.

12. Procédé de fabrication d'une matière colorante pour application cosmétique suivant l'une des revendications 1 à 11, comprenant les étapes suivantes :
- on prépare au préalable un agent effervescent hydrosoluble par mélange d'acide citrique et de carbonate de sodium sous forme cristalline, dans les proportions stoechiométriques pour donner lieu à un dégagement effervescent de dioxyde de carbone en présence d'eau,
- on mélange à sec des pigments avec l'agent effervescent, un agent dispersant choisi parmi les alcools gras éthoxylés et du sorbitol,
- on chauffe ce mélange à une température voisine de 100°C, pour fondre le sorbitol,
- puis on laisse refroidir l'ensemble en le soumettant à une agitation modérée jusqu'à complet refroidissement pour éviter l'agglomération en blocs,
- on tamise les granulés obtenus à une granulométrie adaptée pour leur utilisation à la préparation de produits cosmétiques.

## Patentansprüche

1. Färbende Substanz zur kosmetischen Anwendung, vorliegend in Form von bei gewöhnlicher Temperatur festem Granulat, auf der Basis von pulverförmigen Pigmenten, die mit einem organischen Bindemittel überzogen sind, das aus Sorbitol besteht, welches im Verhältnis von 1 bis 20 Gewichtsprozent bei 60 bis 95 Gewichtsprozent an Pigmenten in der Gesamtgewichtszusammensetzung vorhanden ist, wobei diese darüber hinaus in stöchiometrischen Verhältnissen die Bestandteile eines in Gegenwart von Wasser schäumenden Mittels und einen ethoxilierten Fettalkohol als Dispergiermittel enthält, so dass diese färbende Substanz in wässrigem Milieu dispergierbar ist.

2. Färbende Substanz nach Anspruch 1, **dadurch gekennzeichnet, dass** die Zusammensetzung 1 bis 20 Gewichtsprozent an Schäumungsmittel in Bezug auf das Gesamtgewicht des Materials und 0,1 bis 20 Gewichtsprozent an Dispergiermittel in Bezug auf sein Gesamtgewicht umfasst.

3. Färbende Substanz nach einem der vorhergehenden Ansprüche, **dadurch gekennzeichnet, dass** es 70 bis 90 Gewichtsprozent an Pigmenten in Bezug auf das Gesamtgewicht des Materials und 2 bis 10 Gewichtsprozent Sorbitol, vorzugsweise 4 bis 8 Gewichtsprozent, in Bezug auf das Gesamtgewicht umfasst.

4. Färbende Substanz nach einem der vorhergehenden Ansprüche, **dadurch gekennzeichnet, dass** die Zusammensetzung 5 bis 15 Gewichtsprozent an Schäumungsmittel, insbesondere 8 bis 12 Gewichtsprozent, in Bezug auf das Gesamtgewicht des Materials und 0,5 bis 10 Gewichtsprozent an Dispergiermittel, vorzugsweise 1 bis 5 Gewichtsprozent, in Bezug auf das Gesamtgewicht umfasst.

5. Färbende Substanz nach einem der vorhergehenden Ansprüche, **dadurch gekennzeichnet, dass** es sich bei den Pigmenten um mineralische Pigmente handelt, die unter den Eisenoxiden, Titandioxid, den Interferenzpigmenten Titan-Glimmeroxid und ihren Mischungen in pulvriger Form mit submikronischer Korngröße ausgewählt werden.

6. Färbende Substanz nach einem der vorhergehenden Ansprüche, **dadurch gekennzeichnet, dass** das Schäumungsmittel eine Mischung aus Citronensäure und Natrium(bi)carbonat in stöchiometrischen Verhältnissen ist, um in Gegenwart von Wasser zu einer schäumenden Freisetzung von Kohlendioxid zu führen.

7. Färbende Substanz nach einem der vorangehenden Ansprüche, **dadurch gekennzeichnet, dass** das Dispergiermittel unter den ethoxilierten Fettalkoholen gewählt wird, vorzugsweise unter den ethoxilierten Fettalkoholverbindungen, in deren Kohlenstoffkette sich 8 bis 24 Kohlenstoffatome befinden und auch vorzugsweise 12 bis 20 Kohlenstoffatome in einer verzweigten Kette, und insbesondere unter den ethoxilierten Fettalkoholen, deren Molekül 20 bis 30 oxyethylenierte Funktionen aufweist.

8. Färbende Substanz nach dem vorhergehenden Anspruch, **dadurch gekennzeichnet, dass** der ethoxilierte Fettalkohol ein oxyethylenierter Ether des Cetylstearylalohols ist und insbesondere 25 Ethoxy-Gruppen aufweist.

9. Färbende Substanz nach einem der vorhergehenden Ansprüche, **dadurch gekennzeichnet, dass** es bei gewöhnlicher Temperatur in Form von festem Granulat mit einer mittleren Größe zwischen 0,1 und 5 mm, vorzugsweise zwischen 0,5 und 2 mm, vorliegt.

10. Färbende Substanz nach dem vorhergehenden Anspruch, die einer Zusammensetzung entspricht, die einen Pigmentgehalt zwischen 70 und 90 %, einen Sorbitolanteil zwischen 4 und 8 %, einen Schäumungsmittelanteil zwischen 8 und 12 % und einen Dispergiermittelanteil zwischen 1 und 5 % umfasst.

11. Verwendung einer färbenden Substanz nach einem der Ansprüche 1 bis 10 zur Herstellung von Zusammensetzungen kosmetischer Erzeugnisse durch Einbindung des Materials in die Zusammensetzungen in wässrigem Milieu.

12. Verfahren zur Herstellung einer färbenden Substanz zur kosmetischen Anwendung nach einem der Ansprüche 1 bis 11, das die folgenden Schritte umfasst:
- zunächst wird durch Mischen von Citronensäure und Natriumcarbonat in kristalliner Form in stöchiometrischen Verhältnissen ein wasserlösliches Schäumungsmittel gebildet, so dass es in Gegenwart von Wasser zur schäumenden Freisetzung von Kohlendioxid führt,
- die Pigmente werden im trockenen Zustand mit dem Schäumungsmittel, einem unter den ethoxilierten Fettalkoholen gewählten Dispergiermittel und Sorbitol vermischt,
- diese Mischung wird auf eine Temperatur nahe 100°C erwärmt, um das Sorbitol zu schmelzen,
- dann lässt man das Ganze abkühlen wobei es bis zum vollständigen Abkühlen mäßig stark gerührt wird, um ein Verklumpen zu verhindern,
- das erhaltene Granulat wird durch ein Sieb gegeben, damit es eine Korngröße erhält, die für eine Verwendung bei der Zubereitung von kosmetischen Erzeugnissen geeignet ist.

## Claims

1. Colouring material for cosmetic use which is in the form of granules which are solid at room temperature and is based on pulverulent coated pigments in an organic binder comprising sorbitol present in the proportion of from 1 to 20 % by weight for 60 to 95 % by weight of pigments in the total weight of its composition, this also comprising constituents in stoichiometric proportions of an agent which is effervescent in the presence of water and of an ethoxylated fatty alcohol as a dispersing agent such that the said colouring material is dispersible in an aqueous medium.

2. Colouring material according to claim 1, **characterized in that** the composition comprises from 1 to 20 % by weight of the effervescent agent, based on the total weight of the material, and from 0.1 to 20 % by weight of the dispersing agent, based on its total weight.

3. Colouring material according to one of the preceding claims, **characterized in that** it comprises from 70 to 90 % by weight of pigments, based on the total weight of the material, and from 2 to 10 %, preferably from 4 to 8 % by weight of sorbitol, based on the total weight.

4. Colouring material according to one of the preceding claims, **characterized in that** the composition comprises from 5 to 15 %, in particular from 8 to 12 % by weight of the effervescent agent, based on the total weight of the material, and from 0.5 to 10 %, preferably 1 to 5 % by weight of the dispersing agent, based on the total weight of the material.

5. Colouring material according to one of the preceding claims, **characterized in that** the pigments are mineral pigments chosen from iron oxides, titanium dioxide, mica/titanium oxide interference pigments and their mixtures, in a pulverulent form of submicron particle size.

6. Colouring material according to one of the preceding claims, **characterized in that** the effervescent agent is a mixture of citric acid and sodium (bi)carbonate in stoichiometric proportions in order to give rise to an effervescent release of carbon dioxide in the presence of water.

7. Colouring material according to one of the preceding claims, **characterized in that** the dispersing agent is chosen from ethoxylated fatty alcohols, preferably from ethoxylated compounds of fatty alcohols of which the carbon chain has from 8 to 24 carbon atoms, and more preferably from 12 to 20 carbon atoms, in a branched chain, and more particularly from ethoxylated fatty alcohols of which the molecule comprises from 20 to 30 oxyethylenated functions.

8. Colouring material according to the preceding claim, **characterized in that** the ethoxylated fatty alcohol is an oxyethylenated ether of cetearyl alcohol, in particular comprising 25 ethoxy groups.

9. Colouring material according to one of the preceding claims, **characterized in that** it is in the form of granules which are solid at room temperature, the average size of which is between 0.1 and 5 mm, preferably between 0.5 and 2 mm.

10. Colouring material according to the preceding claim, corresponding to a composition comprising a content of pigment of between 70 and 90 %, a proportion of sorbitol of between 4 and 8 %, a proportion of the effervescent agent of between 8 and 12 % and a proportion of the dispersing agent of between 1 and 5 o.

11. Use of a colouring material according to any one of claims 1 to 10 for the production of cosmetic products by incorporation of the said material into the said compositions in an aqueous medium.

12. Process for the production of a colouring material for cosmetic use according to one of claims 1 to 11, comprising the following stages:
- a water-soluble effervescent agent is first prepared by mixing citric acid and sodium carbonate in the crystalline form in stoichiometric proportions in order to give rise to an effervescent release of carbon dioxide in the presence of water,
- pigments are mixed in the dry state with the effervescent agent, a dispersing agent chosen from the ethoxylated fatty alcohols and sorbitol,
- this mixture is heated to a temperature of about 100 °C in order to melt the sorbitol,
- the entire mixture is then allowed to cool with moderate stirring until cooling is complete, in order to prevent agglomeration in lumps,
- the granules obtained are sieved to a particle size suitable for their use in the preparation of cosmetic products.
